# EUROPEAN PATENT SPECIFICATION

(11) **EP 2 021 459 B1**
(45) Date of publication and mention of the grant of the patent: **27.12.2017**
(21) Application number: 06815465.7
(22) Date of filing: 27.09.2006
(51) Int. Cl.: C12N 5/00, C12M 1/02

(54) **A METHOD TO INCREASE DISSOLVED OXYGEN IN A CULTURE VESSEL**
VERFAHREN ZUR ERHÖHUNG VON GELÖSTEM SAUERSTOFF IN EINEM KULTURGEFÄSS
PROCEDE DE FABRICATION DE BIOREACTEURS EFFICACES

(30) Priority: 08.06.2006 WO PCT/US2006/022312
(43) Date of publication of application: 11.02.2009
(73) Proprietor: ZHEJIANG JINYISHENGSHI BIOENINEERING CO., LTD., Huzhou City Zhejiang 313000 (CN)
(72) Inventor: HUI, Mizhou, Thousand Oaks, CA 91360 (US)
(74) Representative: Chajmowicz, Marion
(86) International application number: PCT/US2006/037468
(87) International publication number: WO 2007/142664

(56) References cited:
- WO-A-2006/138143
- WO-A1-02/42409
- US-A- 4 824 787
- US-A- 4 912 048
- US-A- 5 002 890
- US-A- 5 330 908
- US-A- 5 665 594
- US-A1- 2002 025 547
- US-A1- 2005 106 045
- US-A1- 2010 190 245
- US-B1- 6 391 638
- US-B1- 6 991 933
- MULLER N ET AL: "Orbital shaker technology for the cultivation of mammalian cells in suspension" BIOTECHNOLOGY AND BIOENGINEERING 20050220 JOHN WILEY AND SONS INC. US, vol. 89, no. 4, 20 February 2005 (2005-02-20), pages 400-406, XP009116970
- KATO Y ET AL: JOURNAL OF CHEMICAL ENGINEERING OF JAPAN, vol. 38, no. 11, 2005, pages 873-877, XP009116967
- VIJAY SINGH: "Disposable bioreactor for cell culture using wave-induced agitation" CYTOTECHNOLOGY, KLUWER ACADEMIC PUBLISHERS, DO, vol. 30, no. 1-3, 1 May 1999 (1999-05-01), pages 149-158, XP019236621 ISSN: 1573-0778
- MANO T ET AL: "COMPARISON OF OXYGEN SUPPLY METHODS FOR CULTURES OF SHEAR-STRESS SENSITIVE ORGANISMS INCLUDING ANIMAL CELL CULTURE" JOURNAL OF CHEMICAL TECHNOLOGY AND BIOTECHNOLOGY, BLACKWELL SCIENTIFIC PUBLICATIONS. OXFORD, GB, vol. 47, no. 3, 1 January 1990 (1990-01-01), pages 259-271, XP009013684 ISSN: 0268-2575
- VARLEY J ET AL: "Reactor design for large scale suspension animal cell culture" CYTOTECHNOLOGY, KLUWER ACADEMIC PUBLISHERS, DO, vol. 29, no. 3, 1 March 1999 (1999-03-01), pages 177-205, XP019236596 ISSN: 1573-0778
- ZHANG X ET AL: "Shaken helical track bioreactors: Providing oxygen to high-density cultures of mammalian cells at volumes up to 1000L by surface aeration with air" NEW BIOTECHNOLOGY,, vol. 25, no. 1, 1 June 2008 (2008-06-01), pages 68-75, XP022684678 ISSN: 1871-6784 [retrieved on 2008-05-23]

## Description

### RELATED APPLICATION

This application is the continuation of patent application PCT/US06/22312 entitled "suspension culture vessels," filed on June 08, 2006.

### FIELD OF THE INVENTION

Providing a method to make effective bioreactors is described.

### BACKGROUND OF THE INVENTION

In our previous discovery described in a patent application PCT/US06/22312 entitled "suspension culture vessels," we had described a wide-body culture vessel with an inverted frusto-conical bottom on orbital shaker platform for suspension mammalian cell culture. Surprisingly, this system was significantly better than classical bioreactor and flat bottom shaker bottles. We had described this system making the culture medium climbing up onto the wall of the vessel easily with less hydro-mechanical stress. This system created a broad thin culture medium layer for extended surface, greater aeration and better mixing.

Interestingly, we did not know exact mechanism of action of this shaker-based frusto-conical bottom vessel system. We have now discovered the mechanism of action. Basing on the mechanism of action, namely a method to increase dissolved oxygen level in culture medium, we have designed and tested several types of mammalian cell culture bioreactors.

### SUMMARY OF INVENTION

Mechanism of action of previously described suspension culture vessels with an inversed frusto-conical or inverted frustum bottom (patent application PCT/US06/22312) is disclosed. Providing a method to increase dissolved oxygen (DO) in culture medium, which forms a foundation to design and make effective mammalian cell culture bioreactors, is disclosed.

The invention relates to a method of making air or oxygen microscopic bubbles in a culture medium as recited in claim 1. The invention also relates to a method for oxygenating a culture medium as recited in claim 2. The invention also deals with cell culture apparatus and device as recited in claims 7,8 and 15 and with a method for culturing mammalian cells in suspension as recited in claim 10.

### BRIEF DESCRIPTION OF DRAWINGS

Figure 1 A wide-body vessel with inverted frusto-conical bottom for suspension mammalian cell culture.
Figure 2a Illustration of Flurometrix DO/pH patch sensor detection technology.
Figure 2b Fluorometrix DO/pH patch sensor detection system.
Figure 3 150 ml work volume culture vessel with inverted frusto-conical bottom on shaker platform.
Figure 4 Use of air pump to bubble the culture medium at static status to increase DO level.
Figure 5a, b, c, d, e Nikon digital camera captured instant medium surface characteristics. At an instant moment, all pictures showed titled medium surface level mostly on one side of vessel wall. This characteristics of the medium movement increases DO in the culture medium by repetitively "sweeping" or washing air-exposed smooth vessel surface.
Figure 6a, b Using rolling motion of titled plastic tubes, the culture medium inside the tube repetitively "sweeps" or washes the air-exposed smooth vessel wall surface. This movement increases DO in the medium rapidly to 100%.
Figure 7 Use of plastic tubes with inverted frusto-conical bottom (diameter 3cm), suspension cultured CHOK cells easily reached 2.2% pcv in 4-days of culture on adjustable shaker platform with constant DO 100%. This created an effective mini-bioreactor system for cell clone robustness screening.
Figure 8a A ball-shaped self-rolling bioreactor with back and forth movement for culture medium mixing.
Figure 8b A ball-shaped self-rolling bioreactor on orbital shaker platform for culture medium mixing.
Figure 8c A cone-shaped self-rolling bioreactor vessel with inside projected orbital rails.
Figure 9a 10-liter vessel base with inverted frusto-conical bottom for plastic culture bag.
Figure 9b 10-liter vessel base with inverted frusto-conical bottom.
Figure 10a current Flurometrix cell clone robustness screening and process optimization high-throughput mini-bioreactor system.
Figure 10b Shaker-based multiple wells with frusto-conical bottom for cell line robustness screening.

### DETAILED DESCRIPTION OF THE INVENTION

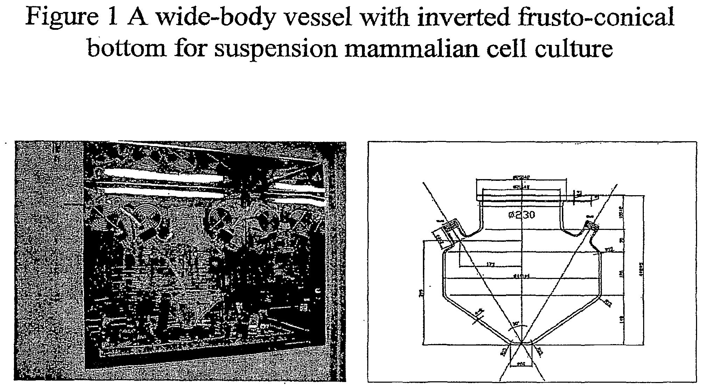

This invention is based, at least in part, on the previous discovery that, without using sophisticated control tower and related DO and pH probes, suspension adapted mammalian cells grew significantly better in culture vessels with an inversed frusto-conical or inverted frustum bottom on a shaker platform with certain motion length than classical Applikon bioreactor as well as flat-bottom shaker bottles (Figure 1).

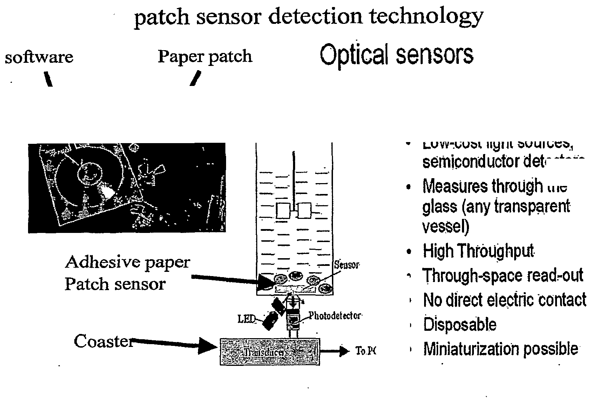

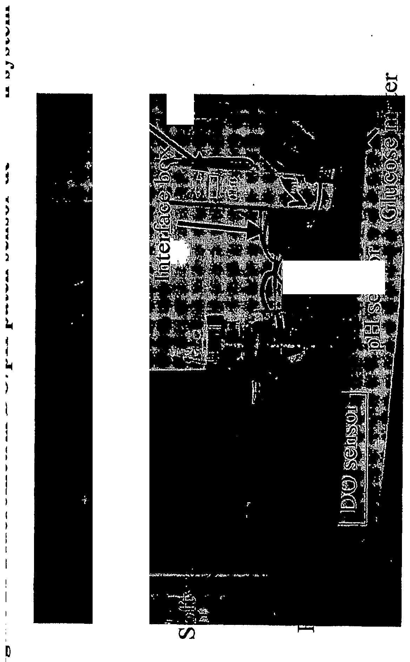

In order to study its mechanism of action, we have employed DO sensor, pH sensor and their detection system (www.flurometrix .com)(Figure 2a, b). We have also employed digital camera (Nikon) to catch and study detailed culture medium movement during shaking motion in the frusto-conical bottom vessels.

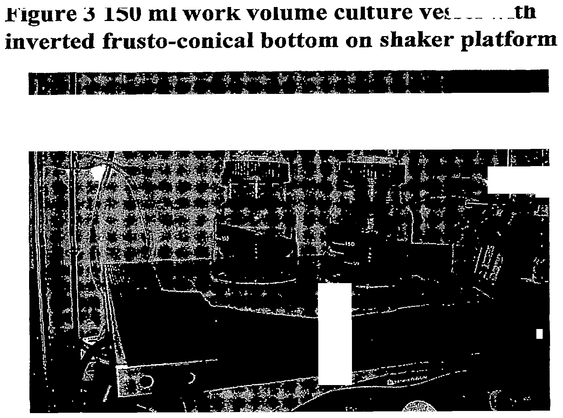

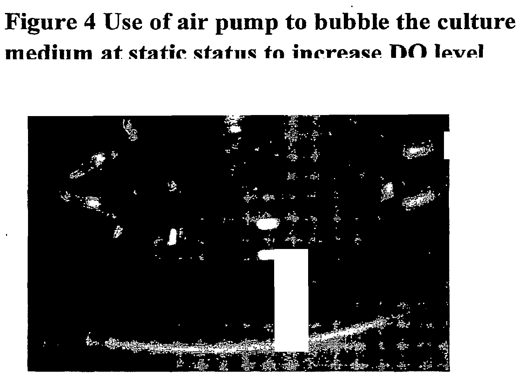

First, we measured DO of the culture medium in 150 ml work volume vessel with inverted conical bottom (Figure 3). We found that DO level easily reached 100% (Table 1). We then used air pump to bubble the culture medium in a same vessel at static status (Figure 4). Surprising it was not able to reach 100% at a reasonable time period (Table 2). We were very surprised by this phenomenon since we routinely used air bubbling method to calibrate DO probe in 3-liter Applikon bioreactor and assumed that DO reached 100%. There must be a mechanism of action behind this phenomenon.

**Table 1 Fresh culture medium was added in 150ml work volume culture vessel with inverted frusto-conical bottom with shaking at 120 rpm (Figure 3). Every 30 minutes, DO was measured.**

| | | | | |
|---|---|---|---|---|
| Time (min) | 0 | 30 | 30 | 30 |
| DO% | 45 | 75 | 92 | 100 |

**Table 2 Fresh culture medium was added in 150ml work volume culture vessel with inverted frusto-conical bottom without shaking. Air pump bubbling was used to add DO into the medium (Figure 4). Every 30 minutes, DO was measured.**

| | | | | |
|---|---|---|---|---|
| Time (min) | 0 | 30 | 30 | 30 |
| DO% | 45 | 52 | 55 | 75 |

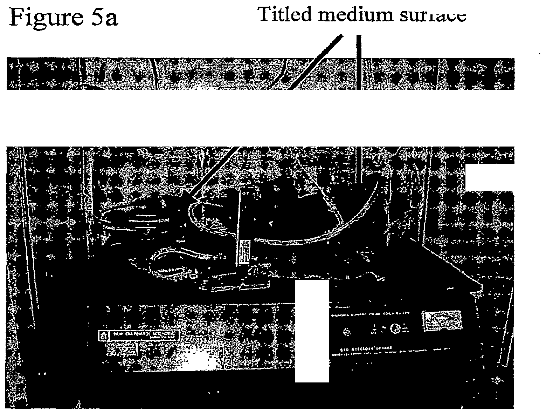

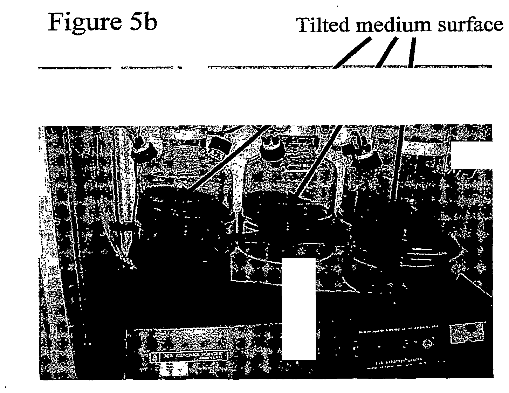

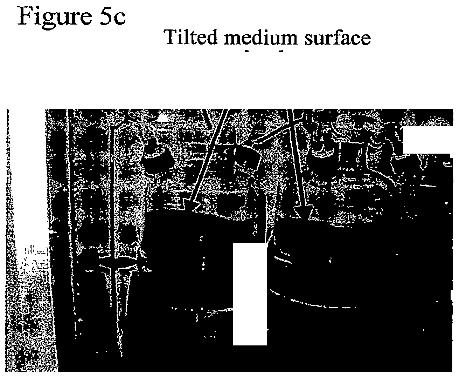

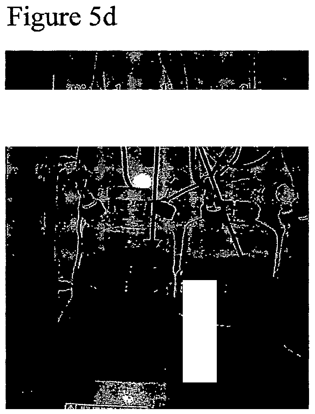

In search of the answer, we used high-speed camera to capture instant movement of the culture medium in the culture vessels with inverted frusto-conical bottom during the shaking (Figure5a, b, c, d)(Figure 3). All the pictures clearly showed that at an instant moment, the culture medium is mostly on one side of culture vessels while most of other side is exposed to air contact. Due to the inverted conical bottom, shaking motion easily move the culture medium climbing onto the one side of vessels wall. This creates a circular movement of the medium current, repeatedly "sweeping" or washing the air exposed vessel wall. We hypothesized that this circular movement and its repetitive "sweeping" increased DO in the culture medium.

Dissolved oxygen (DO) is found in microscopic bubbles of oxygen that are mixed in the water or culture medium and occur between water molecules. In our case of the above, it is possible that tiny oxygen bubbles absorbed on the smooth glass or plastic surface and thus formed a microscopic layer of oxygen bubbles during the instant period of exposure to air. We then hypothesized that the instant formed air bubble layer on the smooth surface are so tiny which resembles the microscopic bubble size of DO in the water. This microscopic bubble layer is then "swept" or washed away by circulating medium current, thus making oxygen dissolved into water easily. This circular movement occurs again and again due to the frusto-conical bottom and shaking motion, thus increasing DO level more efficiently than direct air bubbling into the medium including sparging.

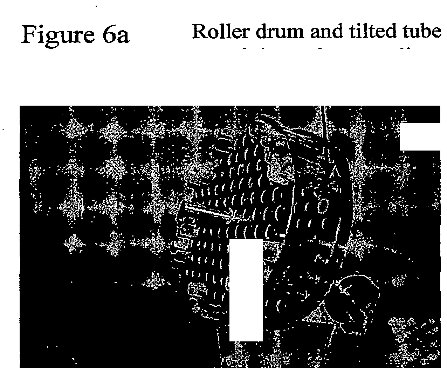

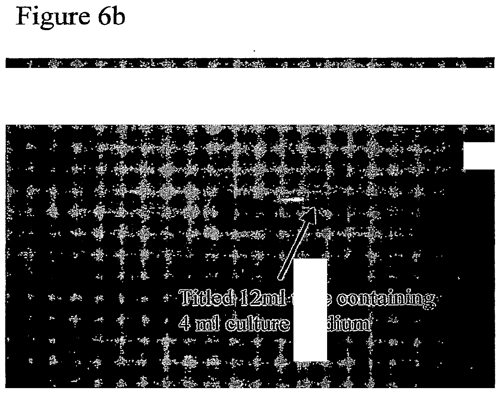

To test this hypothesis, we have employed 12 ml plastic tubes (NUNC) with 4 ml culture medium and roller drum at speed of 60 rpm (Figure 6a, b). Shortly after 10 minutes of rolling, all the medium samples in the tubes have reached 100% DO. This study showed that culture medium or medium current repeatedly sweeping or contacting the air-exposed smooth surface with certain speed or force increased culture medium DO surprisingly effective.

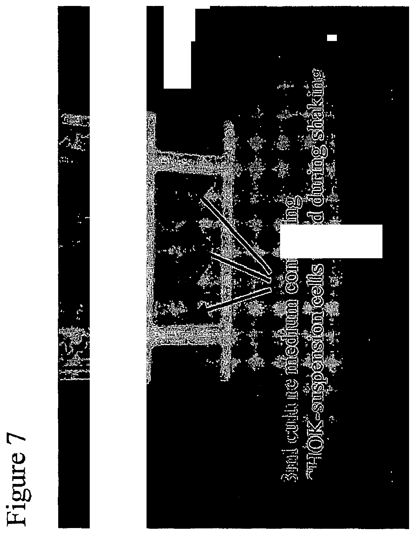

We then cultured CHOK-suspension cells in the tubes on the roller drum at speed of 60 and 100 rpm for 4 days. As expected, DO have reached 100% in all the cases during these 4-day of culture. However, the cells did not grow at all. We thus concluded that there must be need for effective mixing besides of sufficient medium DO for optimal suspension cell culture. We then cultured the cells in 50 ml centrifuge tubes (NUNC) with inverted frusto-conical bottom on an adjustable shaker platform for 4 days (Figure 7). All the cells grew and easily reached 2.2% packed cell volume (pcv). This result indicated that the mixing motion is required besides of sufficient DO for optimal suspension cell culture.

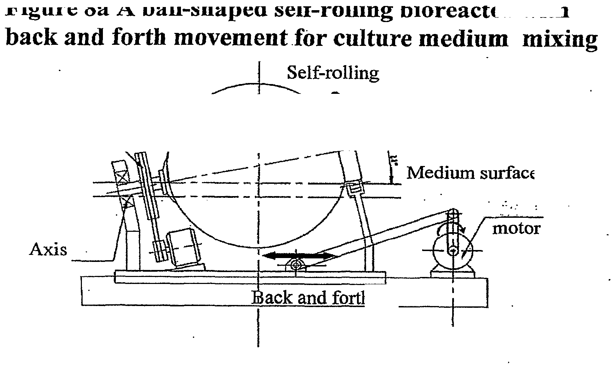

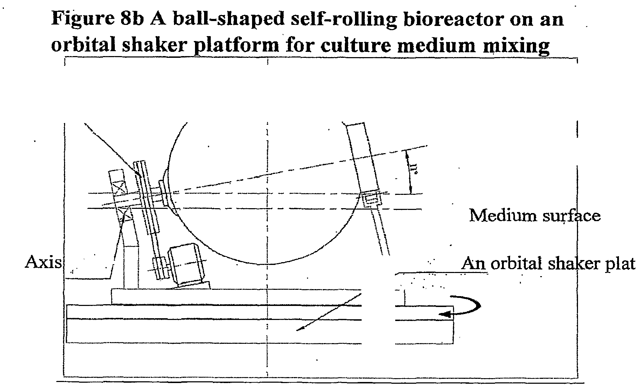

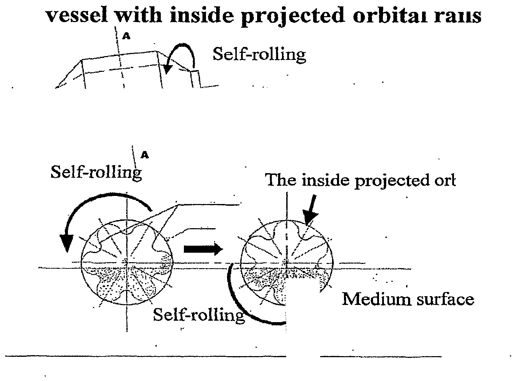

Basing on the above discoveries, we have designed several types of bioreactors for prototype construction. For each type, we have incorporated the method to increase DO in the culture medium by repeatedly using medium current to sweep or contact the air-exposed smooth surface with force together with sufficient medium mixing motion into consideration (Figure 8a,b, c). Details are further described in Example-4.

We have also examined details of a batch-culture process by using a CHOK-suspension cell line expressing TNFR2-Fc-IL-lra in a serum-free suspension culture medium. It was clearly shown that culture vessels with inverted frusto-conical bottom were ideal with optimal DO level, cell density, and yield of the product (Table 3). Details are also described in Example 1.

### Example 1

### Batch culture studying 150ml work volume vessel with inverted frusto-conical bottom

Use of small-scale 150ml work volume shaker vessels for batch culture of CHO production cell line expressing TNFR2-Fc-IL-lra drug candidates was conducted in serum-free culture medium B001 for 8 days. DO was measured every day by using Flurometrix DO patch sensor detection system (Figure 2a, b). Besides use of Flurometrix detection system, pH was also detected by a portable pH meter (Figure 2b). Glucose was measured by a one-touch glucose meter (Figure 2b). Table 3 clearly showed that culture vessels with inverted frusto-conical bottom were ideal with optimal DO level, cell density, and yield of the product.

**Table 3 Simultaneously monitoring DO. pH, glucose. Mixing speed and temperature in a batch culture process in 150ml work volume vessel with inverted frusto-conical bottom.**

| | | | | | | | | |
|---|---|---|---|---|---|---|---|---|
| Day | 1 | 2 | 3 | 4 | 5 | 6 | 7 | 8 |
| DO% | 100 | 100 | 100 | 100 | 100 | 100 | 100 | 100 |
| pH | 7.5 | 7.4 | 7.0 | 6.8 | 6.6 | 6.6 | 6.7 | 6.8 |
| Glucose gram/L | 1.5 | 1.5 | 1.2 | 0.8 | 0.5 | 0.3 | 0.2 | 0.1 |
| Temperature | 37 | 37 | 37 | 34 | 34 | 34 | 34 | 34 |
| Mixing speed rpm | 120 | 120 | 120 | 120 | 120 | 120 | 120 | 120 |
| Expression titer mg/L | 22 | 55 | 115 | 220 | 415 | 530 | 705 | 750 |
| Cell density pcv % | 0.3% | 0.7% | 1.5% | 2.8% | 3.2% | 3.6% | 3.2% | 2.8% |

### Example 2

### Making single-used plastic cell culture bags on bioreactor vessel bases with inverted frusto-conical bottom

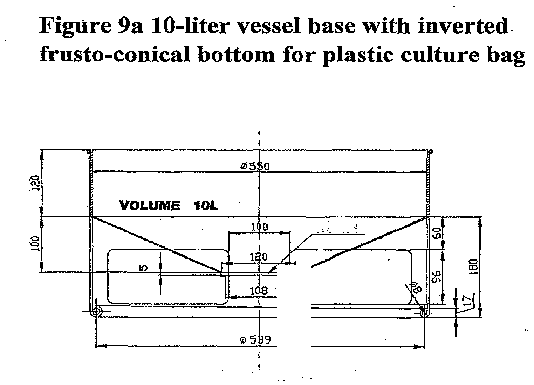

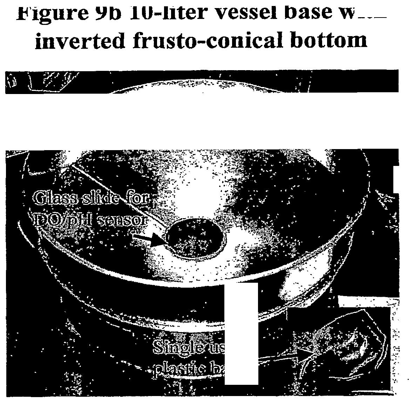

Bioreactor vessel bases with inverted frusto-conical bottom and soft plastic bags (Figure 9a, b) were designed and constructed. These bases and bags were designed to use in shaker platforms with adjustable motion length. The designed frusto-conical bottom together with adjustable shaker platform were intended to make the culture medium climbing as high as possible and as easier as possible (use of minimum shaking energy) to increase DO level in the medium and meet the challenge of high level use of O2 at high cell density culture condition. 3, 10, 20, 40, 100, 500 and 1000-liter size vessel bases and plastic bags have been designed for prototype construction and testing. Our goal is to construct cost-effective shear-force-less single-used mammalian culture bioreactors for R&D and industrial uses.

### Example 3

### Designing shaker-based multiple well plate with inverted frusto-conical bottom for production clone robustness screening after high throughput protein expression titer screening

Robustness of a production cell line is important for stability of scale-up process and ultimate expression yield of a give protein drug. Among the high expression cell lines screened from thousands of cell clones, some of them are robust cell lines who meet industrial production cell standard. The selected robust cell lines are able to grow in high density for longer time and thus generate >10 fold higher expression titer than original screened cell clone expression titer.

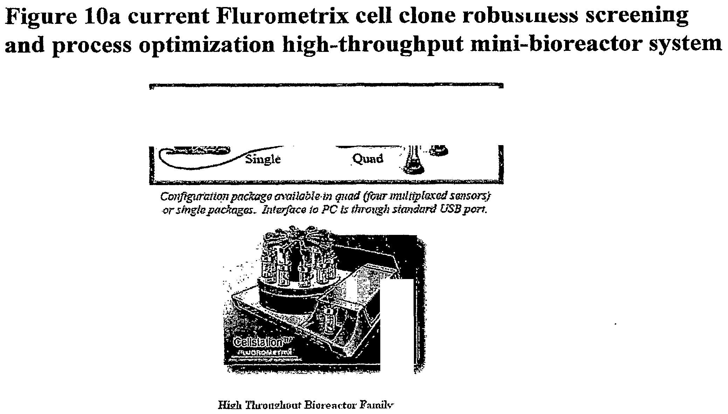

Current mini-bioreactor system (www.flurometrix.com) for cell line robustness screening and process optimization (Figure 10a) is not optimized for high cell density cell growth and does not have optimal DO level to support of high density cell population. Thus it does not have screening of robust cell clones. Without sufficient medium DO, there is no way to optimize fed-batch process at high cell density.

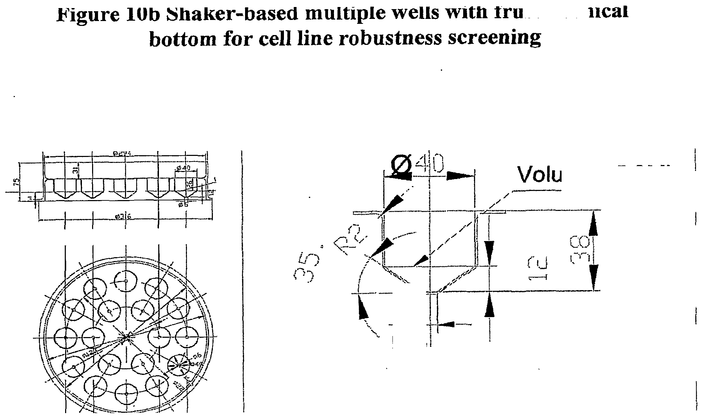

The designed multiple well plate on shaker platform (Figure 10b) will provide sufficient DO in the medium due to shaking motion and frusto-conical bottom of the culture wells to support high cell density growth, thus being able to screen a given cell line's ultimate capacity to grow in highest density and be distinguished from non-robust cell clones. This system is easy to handle and very cost-effective in addition.

### Example 4

### Design of effective bioreactors basing on the method to increase DO in culture medium combined with effective mixing motion

Basing on the above conducted roller drum experiments (Figure 6a, b), we have discovered a method to increase DO in mammalian cell culture medium. We have then designed rolling bioreactors (Figure 8a, b c). Figure 8a shows a ball-shaped self-rolling bioreactor vessel by repetitively washing the air exposed vessel inner surface. This rolling movement increases DO in the culture medium to support high cell density growth. While a back and forth movement at ground level makes the culture medium well mixed during rolling movement (Figure 8a). Together they support optimal suspension cell culture.

Figure 8b shows an ball-shaped self-rolling bioreactor vessel. This rolling movement increases DO in the culture medium by repetitively washing the air exposed vessel inner surface to support high cell density growth. While an orbital shaker-platform at ground level makes the culture medium well mixed during rolling movement. Together they support optimal suspension cell culture.

Figure 8c shows a cone-shaped self-rolling bioreactor vessel. This rolling movement increases DO in the culture medium by repetitively washing the air exposed vessel inner surface to support high cell density growth. While inside projected orbital rails make the culture medium move up to upper one end while rolling and fall back to the lower end. This additional movement helps culture medium mixing during rolling movement. Together they support optimal suspension cell culture.

## Claims

1. A method of making air or oxygen microscopic bubbles in a culture medium, comprising causing a sweeping motion of the medium repetitively across an air-exposed surface, wherein the sweeping motion comprises combining rolling movement with one of the motions chosen among shaking, rocking, and back and forth movement.

2. A method for oxygenating a culture medium comprising :
introducing a culture medium into a culture chamber at least partially filled with gas, wherein the culture chamber is defined by a wall of a vessel, and an inner surface of the wall is configured to promote gas-inner surface contact; and
moving the vessel to create a relative movement of the culture medium with respect to the inner surface of the wall to create microscopic bubbles carrying oxygen molecules in the culture medium, wherein the movement comprises combining rolling movement with one of the motions chosen among shaking, rocking, and back and forth movement.

3. The method of claim 1 or 2, wherein the culture medium is in a culture vessel with an inverted frusto-conical bottom.

4. The method of claim 2, wherein the step of moving the vessel is conducted so that microscopic bubbles carrying oxygen molecules are generated and swept by the moving medium.

5. The method of claim 2, further comprising:
measuring a fluid condition of the culture medium; and
controlling a speed of the relative movement according to the measured fluid condition.

6. The method of claim 5, wherein the fluid condition includes one or more of the following: a pH level, a dissolved oxygen level, and a temperature.

7. A cell culture apparatus comprising:
a rotatable vessel having a ball-shaped wall for defining a culture chamber, the wall having an inner surface configured to promote gas-inner surface contact; and
a motor coupled to the vessel for rotating the vessel about a first axis to increase the level of dissolved oxygen in a medium in the culture chamber, and
further comprising a moveable platform coupled to the rotatable vessel for moving the vessel back and forth, or
further comprising an orbital shaker platform coupled to the rotatable vessel for moving the vessel along a second axis.

8. A cell culture apparatus comprising a rotatable vessel comprising:
an outer casing, and
an inner wall for defining a culture chamber, wherein the inner wall has one or more inside projected rails configured to promote gas-inner surface contact,
a motor coupled to the vessel for rotating the vessel about a first axis to increase the level of dissolved oxygen in a medium in the culture chamber, and
further comprising a moveable platform coupled to the rotatable vessel for moving the vessel back and forth, or
further comprising an orbital shaker platform coupled to the rotatable vessel for moving the vessel along a second axis.

9. The cell culture apparatus of claim 7 or the cell culture apparatus of claim 8, wherein microscopic bubbles carrying oxygen molecules are created in the medium by rotating the vessel.

10. A method for culturing mammalian cells in suspension, wherein the method comprises culturing the cells in a medium in a cell culture apparatus of claim 7 submitted to a back and forth motion, without use of oxygen or air bubbling.

11. A method for culturing mammalian cells in suspension, wherein the method comprises culturing the cells in a medium in a cell culture apparatus of claim 8 with inner projected orbital rails, submitted to a rolling movement, without use of oxygen or air bubbling.

12. The method of claim 10 or 11 wherein the method comprises moving the medium in sweeping motion repetitively across an air-exposed smooth surface of a wall of the vessel.

13. The method of claim 12 wherein the method step is conducted by shaking, rolling, rocking, and flowing.

14. The method of claim 1, 2 or 13, wherein the surface comprises glass, plastic, or metal.

15. A cell culture device comprising :
(1) a cell culture vessel base comprising a chamber that includes (a) a lower section that is inverted frusto-conical bottom or cone-shaped round-bottom configured lower section, and (b) an upper section, and
(2) a cell culture bag that, once filled with a fluid, has a lower part that is inverted frusto-conical bottom or cone-shaped round-bottom configured,
wherein the cell culture bag is detachably disposed within the lower section of the cell culture vessel base.

16. The cell culture device of claim 15, wherein the cell culture bag is disposable.

17. The cell culture device of claim 15, wherein the cell culture vessel base comprises a glass slide for a DO/pH sensor.

18. The cell culture device of claim 15, wherein the lower section and upper section are concentric.

19. An inflatable cell culture bag that, once filled with a fluid, has a lower part that is inverted frusto-conical bottom or cone-shaped round-bottom configured.

## Patentansprüche

1. Ein Verfahren zur Herstellung von mikroskopisch kleinen Luft- oder Sauerstoffblasen in einem Kulturmedium umfassend Verursachen einer schwungvollen Bewegung des Mediums wiederholt über einer Luft-exponierten Oberfläche, wobei die schwungvolle Bewegung das Kombinieren einer rollenden Bewegung mit einer der Bewegungen ausgewählt aus Schütteln, Schaukeln und Hin-und-Her-Bewegung umfasst.

2. Ein Verfahren zur Sauerstoffanreicherung eines Kulturmediums umfassend:
Einführen eines Kulturmediums in eine Kulturkammer, die mindestens teilweise mit Gas gefüllt ist, wobei die Kulturkammer durch eine Wand eines Gefäßes definiert ist und eine innere Oberfläche der Wand ist konfiguriert, um den Gas-innere Oberfläche Kontakt zu fördern, und
Bewegen des Gefäßes um eine relative Bewegung des Kulturmediums gegenüber der inneren Oberfläche der Wand zu schaffen, um mikroskopisch kleine Blasen, die Sauerstoffmoleküle im Kulturmedium tragen, zu erzeugen, wobei die Bewegung das Kombinieren einer rollenden Bewegung mit einer der Bewegungen ausgewählt aus Schütteln, Schaukeln und Hin-und-Her-Bewegung umfasst.

3. Das Verfahren nach Anspruch 1 oder 2, wobei das Kulturmedium in einem Kulturgefäß mit einem umgekehrt kegelstumpfförmigen Boden ist.

4. Das Verfahren nach Anspruch 2, wobei der Schritt des Bewegens des Gefäßes so ausgeführt wird, dass mikroskopisch kleine Blasen, die Sauerstoffmoleküle tragen, generiert werden und umgeben sind von dem sich bewegenden Medium.

5. Das Verfahren nach Anspruch 2, ferner umfassend:
Messen eines Flüssigkeitszustands des Kulturmediums und
Kontrollieren einer Geschwindigkeit der relativen Bewegung gemäß des gemessenen Flüs sigkeitszustands.

6. Das Verfahren nach Anspruch 5, wobei der Flüssigkeitszustand ein oder mehr der folgenden beinhalten: ein pH Level, ein Gelöstsauerstoffgehalt und eine Temperatur.

7. Eine Zellkulturvorrichtung umfassend:
ein rotierbares Gefäß, das ein kugelförmige Wand zum Definieren einer Kulturkammer aufweist, die Wand hat eine innere Oberfläche, die konfiguriert ist, um den Gas-innere Oberfläche Kontakt zu fördern und
einen Motor, der an das Gefäß gekoppelt ist zum Rotieren des Gefäßes um eine erste Achse, um den Level des Gelöstsauerstoffs in einem Medium in der Kulturkammer zu erhöhen und
ferner umfassend eine bewegliche Plattform, die an das rotierbare Gefäß gekoppelt ist zur Hin- und-Her-Bewegung des Gefäßes oder ferner umfassend eine Orbitalschüttler-Plattform,
die an das rotierbare Gefäß zur Bewegung des Gefäßes entlang einer zweiten Achse gekoppelt ist.

8. Eine Zellkulturvorrichtung umfassend ein rotierbares Gefäß umfassend:
ein Außengehäuse und
eine innere Wand zum Definieren einer Kulturkammer, wobei die innere Wand eine oder
mehr innen-projizierte Bahnen aufweist, die konfiguriert sind um den Gas-innere Oberflächen-Kontakt zu fördern,
einen Motor, der an das Gefäß gekoppelt ist zum Rotieren des Gefäßes um eine erste Achse, um den Level des Gelöstsauerstoffs in einem Medium in der Kulturkammer zu erhöhen und
ferner umfassend eine bewegliche Plattform, die an das rotierbare Gefäß gekoppelt ist zur Hin- und-Her-Bewegung des Gefäßes oder ferner umfassend eine Orbitalschüttler-Plattform,
die an das rotierbare Gefäß zur Bewegung des Gefäßes entlang einer zweiten Achse gekoppelt ist.

9. Die Zellkulturvorrichtung nach Anspruch 7, oder die Zellkulturvorrichtung nach Anspruch 8, wobei mikroskopisch kleine Blasen, die Sauerstoffmoleküle tragen durch Rotieren des Gefäßes in dem Medium gebildet werden.

10. Ein Verfahren zur Kultivierung von Säugetierzellen in Suspension, wobei das Verfahren das Kultivieren der Zellen in einem Medium in einer Zellkulturvorrichtung nach Anspruch 7 umfasst, die einer Hin-und-Her-Bewegung ohne Verwendung von Sauerstoff oder Luftblasen unterworfen werden.

11. Ein Verfahren zur Kultivierung von Säugetierzellen in Suspension, wobei das Verfahren das Kultivieren der Zellen in einem Medium in einer Zellkulturvorrichtung nach Anspruch 8 umfasst mit innen-projizierten orbitalen Bahnen, die einer rollenden Bewegung ohne Verwendung von Sauerstoff oder Luftblasen unterworfen werden.

12. Das Verfahren nach Anspruch 10 oder 11, wobei das Verfahren das Bewegen des Mediums in einer schwungvollen Bewegung wiederholt über einer Luft-exponierten, glatten Oberfläche einer Wand des Gefäßes umfasst.

13. Das Verfahren nach Anspruch 12, wobei der Verfahrensschritt durch Schütteln, Rollen, Schaukeln und Fließen ausgeführt wird.

14. Das Verfahren nach Anspruch 1, 2 oder 13, wobei die Oberfläche Glas, Kunststoff oder Metall umfasst.

15. Eine Zellkulturvorrichtung umfassend:
(1) ein Zellkulturgefäßboden umfassend eine Kammer die (a) einen unteren Abschnitt, der ein umgekehrt kegelstumpfförmiger Boden- oder ein kegelförmiger Rundboden-konfigurierter unterer Abschnitt ist und (b) einen oberen Abschnitt umfasst und
(2) eine Zellkulturtasche, die nach dem Befüllen mit einer Flüssigkeit, einen unteren Bereich aufweist, der umgekehrt kegelstumpfförmig Boden- oder kegelförmig Boden-konfiguriert ist, wobei die Zellkulturtasche innerhalb des unteren Abschnitts des Zellkulturgefäßbodens abnehmbar angeordnet ist.

16. Die Zellkulturvorrichtung nach Anspruch 15, wobei die Zellkulturtasche abnehmbar ist.

17. Die Zellkulturvorrichtung nach Anspruch 15, wobei der Zellkulturgefäßboden eine Glasfolie für einen DO/pH Sensor umfasst.

18. Die Zellkulturvorrichtung nach Anspruch 15, wobei der untere Abschnitt und der obere Anschnitt konzentrisch sind.

19. Eine aufblasbare Zellkulturtasche, die nach Befüllen mit einer Flüssigkeit, einen oberen Bereich aufweist, der umgekehrt kegelstumpfförmig Boden- oder kegelförmig Boden-konfiguriert ist.

## Revendications

1. Méthode de production de bulles microscopiques d'air ou d'oxygène dans un milieu de production, comprenant la réalisation d'un mouvement de balayage du milieu de manière répétitive à travers une surface exposée à l'air, dans laquelle le mouvement de balayage comprend la combinaison d'un mouvement de roulement avec l'un des mouvements choisis parmi un secouage, une oscillation, et un mouvement de va et vient.

2. Méthode d'oxygénation d'un milieu de culture comprenant :
l'introduction d'un milieu de culture dans une chambre de culture au moins partiellement remplie de gaz, la chambre de culture étant définie par une paroi d'un récipient, et une surface intérieure de la paroi étant configurée pour favoriser un contact entre le gaz et la surface intérieure ; et
le déplacement du récipient pour créer un mouvement relatif du milieu de culture par rapport à la surface intérieure de la paroi pour créer des bulles microscopiques transportant des molécules d'oxygène dans le milieu de culture, le mouvement comprenant une combinaison d'un mouvement de roulement avec l'un des mouvements choisis parmi un secouage, une oscillation, et un mouvement de va et vient.

3. Méthode selon la revendication 1 ou 2, dans laquelle le milieu de culture est dans un récipient de culture avec un fond tronconique inversé.

4. Méthode selon la revendication 2, dans laquelle l'étape de déplacement du récipient est effectuée de façon que des bulles microscopiques transportant des molécules d'oxygène soient générées et balayées par le milieu en mouvement.

5. Méthode selon la revendication 2, comprenant en outre :
la mesure d'une condition de fluide du milieu de culture ; et
le contrôle d'une vitesse du mouvement relatif en fonction de la condition de fluide mesurée.

6. Méthode selon la revendication 5, dans laquelle la condition de fluide comprend un ou plusieurs des éléments suivants : un niveau de pH, un niveau d'oxygène dissous, et une température.

7. Dispositif de culture cellulaire comprenant :
un récipient rotatif ayant une paroi en forme de balle pour définir une chambre de culture, la paroi ayant une surface intérieure configurée pour favoriser un contact entre un gaz et la surface intérieure ; et
un moteur couplé au récipient pour faire tourner le récipient autour d'un premier axe de manière à augmenter le niveau d'oxygène dissous dans un milieu dans la chambre de culture, et
comprenant en outre une plateforme mobile couplée au récipient rotatif pour déplacer le récipient en va et vient, ou
comprenant en outre une plateforme d'agitation orbitale couplée au récipient rotatif pour déplacer le récipient le long d'un deuxième axe.

8. Dispositif de culture cellulaire comprenant un récipient rotatif comprenant :
une enceinte extérieure, et
une paroi intérieure pour définir une chambre de culture, la paroi intérieure ayant un ou plusieurs rails faisant saillie vers l'intérieur configurés pour favoriser un contact entre un gaz et la surface intérieure,
un moteur couplé au récipient pour faire tourner le récipient autour d'un premier axe de manière à augmenter le niveau d'oxygène dissous dans un milieu dans la chambre de culture, et
comprenant en outre une plateforme mobile couplée au récipient rotatif pour déplacer le récipient en va et vient, ou
comprenant en outre une plateforme d'agitation orbitale couplée au récipient rotatif pour déplacer le récipient le long d'un deuxième axe.

9. Dispositif de culture cellulaire selon la revendication 7 ou dispositif de culture cellulaire selon la revendication 8, dans lequel des bulles microscopiques transportant des molécules d'oxygène sont créées dans le milieu par rotation du récipient.

10. Méthode de culture de cellules de mammifère en suspension, laquelle méthode comprend la culture des cellules dans un milieu dans un dispositif de culture cellulaire selon la revendication 7 soumis à un mouvement de va et vient, sans utilisation de barbotage d'oxygène ou d'air.

11. Méthode de culture de cellules de mammifère en suspension, laquelle méthode comprend la culture des cellules dans un milieu dans un dispositif de culture cellulaire selon la revendication 8 avec des rails orbitaux faisant saillie vers l'intérieur, soumis à un mouvement de roulement, sans utilisation de barbotage d'oxygène ou d'air.

12. Méthode selon la revendication 10 ou 11, laquelle méthode comprend le déplacement du milieu selon un mouvement de balayage de manière répétitive à travers une surface lisse exposée à l'air d'une paroi du récipient.

13. Méthode selon la revendication 12, dans laquelle l'étape de méthode est effectuée par secouage, roulement, oscillation, et écoulement.

14. Méthode selon la revendication 1, 2 ou 13, dans laquelle la surface comprend du verre, du plastique, ou du métal.

15. Dispositif de culture cellulaire comprenant :
(1) une base de récipient de culture cellulaire comprenant une chambre qui comprend (a) une section inférieure qui est une section intérieure configurée avec un fond tronconique inversé ou un fond rond en forme de cône, et (b) une section supérieure, et
(2) une poche de culture cellulaire qui, une fois remplie d'un fluide, a une partie inférieure qui est configurée avec un fond tronconique inversé ou un fond rond en forme de cône,
dans lequel la poche de culture cellulaire est disposée de façon détachable à l'intérieur de la section inférieure de la base de récipient de culture cellulaire.

16. Dispositif de culture cellulaire selon la revendication 15, dans lequel la poche de culture cellulaire est jetable.

17. Dispositif de culture cellulaire selon la revendication 15, dans lequel la base de récipient de culture cellulaire comprend une lamelle en verre pour un capteur de DO/pH.

18. Dispositif de culture cellulaire selon la revendication 15, dans lequel la section inférieure et la section supérieure sont concentriques.

19. Poche de culture cellulaire gonflable qui, une fois remplie d'un fluide, a une partie inférieure qui est configurée avec un fond tronconique inversé ou un fond rond en forme de cône.
